(19) Europäisches Patentamt
European Patent Office
Office européen des brevets

(11) Veröffentlichungsnummer: **0 226 942**
**A2**

(12) **EUROPÄISCHE PATENTANMELDUNG**

(21) Anmeldenummer: 86117165.0

(22) Anmeldetag: 09.12.86

(51) Int. Cl.⁴: C 07 C 51/12
C 07 C 57/04, B 01 J 27/13
B 01 J 31/30

(30) Priorität: 18.12.85 DE 3544765

(43) Veröffentlichungstag der Anmeldung:
01.07.87 Patentblatt 87/27

(84) Benannte Vertragsstaaten:
DE FR GB IT NL

(71) Anmelder: Röhm GmbH
Kirschenallee Postfach 4242
D-6100 Darmstadt 1(DE)

(72) Erfinder: Langerbeins, Klaus, Dr. Dipl.-Chem.
Bahnstrasse 31-33
D-6070 Langen(DE)

(54) **Verfahren zur Herstellung von ungesättigten aliphatischen Carbonsäureanhydriden.**

(57) Herstellung von α,β-ungesättigten Carbonsäureanhydriden durch Carbonylierung von Acrylsäure- bzw. Methacrylsäureestern, in Gegenwart von Katalysatorsystemen, die mindestens ein Edelmetall der Gruppe VIII des Periodensystems, z.B. Rhodium, enthalten, und die Umsetzung in Gegenwart von Halogen bzw. Halogenverbindungen, insbesondere Jod bzw. Jodverbindungen, als Promotoren, bei 70 bis 350°C und bei Drücken von 1 bis 500 bar durchgeführt wird.

EP 0 226 942 A2

# Verfahren zur Herstellung von ungesättigten aliphatischen Carbonsäureanhydriden

## Beschreibung

### Gebiet der Erfindung

Die Erfindung betrifft ein Verfahren zur Herstellung von ungesättigten aliphatischen Carbonsäureanhydriden durch Umsetzung von Estern ungesättigter aliphatischer Carbonsäuren mit Kohlenmonoxid. Anhydride ungesättigter Carbonsäuren, insbesondere der Acryl- und Methacrylsäure, sind infolge ihrer Reaktivität wichtige Ausgangsverbindungen für die Herstellung von interessanten, auf anderen Herstellungswegen schwer zugänglichen Monomeren.

### Stand der Technik

Die Herstellung von aliphatischen gesättigten Carbonsäureanhydriden durch Carbonylierung von Carbonsäureestern unter Verwendung von Nickel- und Halogen-haltigen Katalysatoren wird beispielsweise in der US-A 2 729 651 beschrieben. Auch die DE-A 28 44 371 beschreibt ein Verfahren zur Herstellung von Carbonsäureanhydriden aus aliphatischen Carbonsäureestern, worunter nach der Beschreibung auch solche aus ungesättigten aliphatischen Carbonsäuren zu verstehen sind, unter Verwendung von Nickelkatalysatoren. Gegenüber dem Verfahren der US-A kann bei dem Vorgehen nach der DE-A durch das Vorhandensein bestimmter Mengen einer freien Jodverbindung die Carbonylierungsreaktion bei wesentlich niedrigeren Drücken, beispielsweise bei etwa 50 Atmosphären anstelle von etwa 700 Atmosphären, bei sonst üblichen Temperaturen von etwa 70 bis 250°C durchgeführt werden. Durchführungen oder Angaben zu Durchführungen von Carbonylierungen mit ungesättigten aliphatischen Carbonsäureestern, vor allem der Acryl- oder Methacrylsäure, zu ungesättigten Carbonsäureanhydriden sind aus den angegebenen Literaturstellen nicht zu ersehen.

Eigene Versuche zur Carbonylierung von Methylmethacrylat in Gegenwart von Nickelkatalysatoren und Methyljodid ergaben, daß nur geringe Mengen von Methacrylsäureanhydrid entstanden waren. In deutlich größerer Menge wurde dabei das Dimerisationsprodukt von Methylmethacrylat gebildet, das nach DE-A 33 36 691 durch katalytische Dimerisation in Gegenwart von Nickel-Phosphin-Komplex-Katalysatoren entsteht.

Nach einem Verfahren der DE-A 33 32 018 führen Carbonylierungen von Acrylsäureestern in Gegenwart von Alkoholen mit Cobaltcarbonyl-Komplexen als Katalysatoren, d.h. mit Cobalt, das neben Nickel eines der wirksamsten Carbonylierungsmetalle ist und wie dieses zu den Eisenmetallen der 8. Nebengruppe des Periodensystems zählt, zu Bernsteinsäurediestern.

Eine Carbonylierung eines gesättigten Carbonsäureesters, nämlich des Essigsäuremethylesters zu Essigsäureanhydrid, in Gegenwart von Katalysatoren, die Edelmetalle der 8. Nebengruppe des Periodensystems oder deren Verbindungen, sowie Jod und/oder Jodverbindungen enthalten, wird in der DE-A 24 50 965 beschrieben.

Aufgabe und Lösung

Der Erfindung lag daher die Aufgabe zugrunde, für die Herstellung der wertvollen ungesättigten Carbonsäureanhydride von Acrylsäure und Methacrylsäure, ein Verfahren durch Carbonylierung von Estern dieser ungesättigten Säuren zur Verfügung zu stellen.

Es wurde gefunden, daß sich überraschenderweise bei einer katalysierten Carbonylierung von Estern ungesättigter Carbonsäuren die Anhydride der ungesättigten Carbonsäuren bilden.
Gegenstand der Erfindung ist daher ein Verfahren zur Herstellung von ungesättigten aliphatischen Carbonsäureanhydriden, das dadurch gekennzeichnet ist, daß man einen ungesättigten aliphatischen Carbonsäureester mit Kohlenmonoxid in Gegenwart eines Katalysators,

der Edelmetalle der Gruppe VIII des Periodensystems und/oder deren Verbindungen, gegebenenfalls mit Komplexliganden und wahlweise noch weitere Cokatalysatoren enthält, und in Gegenwart von Halogen und/oder Halogenverbindungen, bei Temperaturen von 70 bis 350°C und Drücken von 1 bis 500 bar, umsetzt.

Durch das erfindungsgemäße Verfahren wurde ein nach dem Stand der Technik vorhandenes Vorurteil überwunden, wonach ungesättigte Ester primär und ausschließlich an den ungesättigten Kohlenstoffatomen carbonyliert werden und somit eine Einschiebung des Kohlenmonoxids in die Estergruppierung der ungesättigten Carbonsäureverbindungen zur Bildung von Anhydriden mit Doppelbindungsfunktion unterbleibt.

Vorteile der Erfindung

Nach dem erfindungsgemäßen Verfahren ist es jetzt möglich mit gut zugänglichen und preiswerten Ausgangsstoffen, wie es das Kohlenmonoxid und viele der bekannten Ester, der Acrylsäure oder der Methacrylsäure sind, entsprechende ungesättigte Carbonsäureanhydride preiswert und in ausreichender Menge zur Verfügung zu stellen, was nach bisherigen Verfahren, wie durch Umanhydridisierung der ungesättigten Carbonsäuren mit z.B. Essigsäureanhydrid oder durch Umsetzung ungesättigter Carbonsäurechloride mit Salzen der entsprechenden Säuren, nicht gegeben war.

Bei der erfindungsgemäßen Carbonylierung der ungesättigten Carbonsäureester entstehen zunächst gemischte Anhydride mit der ungesättigten Carbonsäurekomponente des Esters und mit der Carbonsäurekomponente, die durch Einschiebung von CO aus der Alkoholkomponente des Esters entsteht, und die dann eine um ein Kohlenstoffatom längere Kohlenstoffkette als der Esteralkohol enthält.

Durch Reaktionen, die schon während der Carbonylierungsreaktion ablaufen, bilden sich daraus andere Anhydride, die entsprechend Temperatur und katalysierter Beeinflussung dann im Reaktionsgemisch in bestimmten Mengenverhältnissen vorliegen. Bei der Aufarbeitung, insbesondere durch Destillation kann man die verschiedenen Anhydride voneinander trennen. Beispielsweise entsteht bei der erfindungsgemäßen Carbonylierung von Methylmethacrylat zunächst das gemischte Anhydrid aus Methacrylsäure und Essigsäure, aus dem schon während des Carbonylierungsprozesses Methacrylsäureanhydrid und Essigsäureanhydrid in erheblichen Anteilen entstehen. Durch Destillation kann man Essigsäureanhydrid, das gemischt-ungesättigt-gesättigte Anhydrid und das Methacrylsäureanhydrid voneinander trennen. Das gemischte Anhydrid ist ebenfalls ein wertvolles Produkt und kann z.B. als solches für die Durchführung von Reaktionen verwendet werden, oder es kann nach bekannten Methoden praktisch vollständig in das ungesättigte Anhydrid, wie beispielsweise das Methacrylsäureanhydrid, und in das Anhydrid, welches letztlich aus dem Esteralkohol gebildet wird und ebenfalls ein wertvolles Beiprodukt ist, überführt werden.

Ausführung der Erfindung

Als Ausgangsstoffe kommen neben dem Kohlenmonoxid, das nicht vollkommen rein sein muß und inerte Bestandteile wie Stickstoff, Kohlendioxid oder Methan auch in größeren Mengen, z.B. von etwa 50 Vol.-%, und gegebenenfalls geringe Mengen Wasserstoff, enthalten kann, Ester von α,ß-ungesättigten Carbonsäuren der allgemeinen Formel

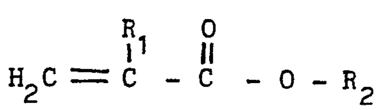

in Frage, worin

$R_1$ = H oder $CH_3$
und
$R_2$ = Alkyl mit 1 bis 10 C-Atomen.
Cycloalkyl mit 5 bis 10 C-Atomen.
Alkenyl mit 2 bis 10 C-Atomen.
Aryl oder Aralkyl.
wobei $R_2$ noch inerte Substituenten enthalten kann,

bedeuten.

Beispiele für geeignete Ester sind danach Methyl-, Äthyl-, Propyl-, i-Butyl-, Amyl-, Decyl-, Cyclohexyl-, Allyl-, Chloräthyl-, Phenyl- oder Benzylester von Acrylsäure und Methacrylsäure. Sehr geeignet sind die großtechnisch zugänglichen Ester der Acrylsäure und der Methacrylsäure, wobei vom wirtschaftlichen Standpunkt insbesondere die niederen Alkylester, vorzugsweise die Methylester für das erfindungsgemäße Vorgehen eingesetzt werden. Erfindungsgemäß wird die Carbonylierung des α,ß-ungesättigten Carbonsäureesters zu dem ungesättigten Carbonsäureanhydrid in Gegenwart eines Katalysators durchgeführt, der als wesentliche Bestandteile Edelmetalle der Gruppe VIII des Periodensystems und/oder deren Verbindungen, sowie Halogen, wie Brom oder Jod bzw. Halogenverbindungen enthält.

Die Edelmetallkomponente des Katalysators umfaßt die Elemente Ruthenium, Rhodium, Palladium, Osmium, Iridium und Platin, wobei diese einzeln oder im Gemisch vorhanden sein können. Sehr aktive Katalysatoren erhält man beispielsweise, wenn man Rhodium, Rhodiumchlorid, oder Palladium oder Platin bzw. deren Verbindungen bei dem erfindungsgemäßen Verfahren einsetzt. Die Form, in welcher die Edelmetalle der Gruppe VIII des Periodensystems eingesetzt werden, kann weitgehend verschieden sein.

So können die Edelmetalle als solche, bevorzugt in Form feinverteilter Metalle, wie sie z.B. als Raney-Rhodium oder Raney-Palladium bekannt sind, oder in Form einfacher Verbindungen, wie beispielsweise $IrCl_3$, $IrBr_3 \cdot 3H_2O$, Ru $O_2$, $OsO_4$, $PdCl_2$, $Rh(NO_3)_3 \cdot 2H_2O$, $Rh_2\ (SO_4)_3$, $RuO_2$, $OsO_4$ oder als Edelmetallcarboxylate wie Rhodium-III-acetat oder Platin-II-acetat eingesetzt werden.
Der aktive katalytische Bestandteil ist vorzugsweise eine Komplexverbindung der Edelmetalle mit beispielsweise Halogen, wie Chlor, Brom und/oder Jod, Hydroxyl, Wasser, Kohlenmonoxid, sowie auch geeigneten Verbindungen des 3-wertigen Stickstoffs und Organophosphinverbindungen, wie beispielsweise Tributylphosphin oder Triphenylphosphin, als Liganden. Auch in Form von z.B. Metallcarbonylen, Metallcarbonylhalogeniden werden die erfindungsgemäßen Katalysatoren angewendet.

Auch die Halogen-haltige Katalysatorkomponente kann von der Art der eingesetzten Halogenverbindung her verschiedenster Art sein. Am wirksamsten sind Brom und/oder Jod, die insbesondere in Form von Alkylhalogeniden, vorzugsweise Alkyljodide wie Methyljodid oder als Acyljodide eingesetzt werden. Aber auch die Alkalihalogenide wie NaBr, NaJ, KBr, KJ oder die Ammoniumsalze, sowie entsprechende quartäre Ammonium- oder Phosphoniumverbindungen kommen als Katalysatorkomponenten in Betracht.

Promotoren, die dem Katalysatorsystem noch zugesetzt werden, und die wie die Halogenkomponenten in erheblichem molarem Überschuß bezogen auf die Edelmetallkomponente vorhanden sein können, sind die schon erwähnten 3-wertigen Stickstoffverbindungen, wie Pyridine, Alkylamine, Aniline, die 3-wertigen Phosphorverbindungen, wie Alkyl- oder Aryl- oder gemischte Alkyl-aryl-phosphine oder 3-wertige Arsenverbindungen, wie z.B. Triphenylarsen oder analoge Antimonverbindungen, sowie andere bekannte organische komplexbildende Verbindungen wie z.B. Lactone, beispielsweise Butyrolactan.

Daneben zeigen auch noch andere Metalle bzw. Metallverbindungen, wie z.B. der carbonylbildenden Metalle Cr, Mo, W, Fe, Co oder Ni oder andere Metalle bzw. Metallverbindungen wie z.B. $VCl_3$ oder $AlCl_3$ gute Promotorwirkung.

Die Katalysatoren können in gelöster oder in fester Form, z.B. als feinverteilte Suspension, eingesetzt werden. Auch auf üblichen Trägermaterialien, wie z.B. Kieselgur, Aluminiumoxid, oder Aktivkohle aufgebracht, können feste Katalysatoren, z.B. auch als Festbett, angewendet werden. Als Lösungsmittel für Katalysatoren und als Reaktionsmedium dienen die üblicherweise flüssigen ungesättigten Carbonsäureester. In den Fällen, in denen unter den Arbeitsbedingungen nicht flüssige ungesättigte Carbonsäureester zum Einsatz kommen, wird die Umsetzung in inerten Lösungsmitteln, wie beispielsweise Tetrachlorkohlenstoff, Chlorbenzol, Hexan, Heptan oder Cyclohexan durchgeführt.

Im Katalysatorsystem werden im allgemeinen Verhältnisse von Promotorkomponenten zur aktiven Edelmetallkomponente verwendet, die bei 1:1 bis 2000:1, besonders 2:1 bis 1000:1, bevorzugt 3:1 bis 500:1 Mole Promotor pro Atom Edelmetall liegen. Die Menge des Katalysatorsystems, bezogen auf den eingesetzten ungesättigten Carbonsäureester liegt bei 0,01 bis 50 Gew.-%, besonders bei 0,05 bis 40 Gew.-%, bevorzugt bei 0,1 bis 25 Gew.-%.

Die Reaktion wird im Bereich von 70 bis 350°C ausgeführt. Bevorzugt arbeitet man im Bereich von 100 bis 250°C. Bei der Umsetzung können Drücke von 1 bis 500 bar, vorzugsweise solche von 1 bis 250 bar und besonders bevorzugt solche von 10 bis 150 bar angewendet werden.

Nach der Aufarbeitung, z.B. durch Filtration und/oder Destillation, kann der dabei anfallende Rückstand, der u.a. das eingesetzte Edelmetall in praktisch quantitativer Menge enthält, zusammen mit

nicht umgesetztem Ausgangsester und zurückgewonnenen Promotoren, wie z.B. das Methyljodid, wieder in eine neue Umsetzung eingegeben werden.

Das neue Herstellungsverfahren läßt sich sowohl kontinuierlich als auch diskontinuierlich betreiben. Bei einer großtechnischen Ausgestaltung wird man jedoch vorzugsweise die Reaktion kontinuierlich durchführen, wobei sowohl mit gelöstem und/oder suspendiertem Katalysator oder mit festem Katalysatorbett gearbeitet werden kann.

Zur Vermeidung unerwünschter Polymerisationen ist es zweckmäßig, das erfindungsgemäße Carbonylierungsverfahren in Gegenwart wirksamer Mengen von Polymerisationsinhibitoren wie Hydrochinon, Hydrochinonmonomethyläther, Phenothiazin oder Kupferverbindugnen durchzuführen.

Beispiele

Die Stabilisierung aller Ansätze erfolgt mit 100 ppm Kupferoleat.

Beispiel 1

Ein Gemisch aus 547,0 mmol Methylmethacrylat, 1,0 mmol Rhodium(III)-chlorid ($RhCl_3$ x 3,5 $H_2O$), 50,8 mmol Triphenylphosphin und 100,0 mmol Methyljodid wird in einen 0,35-l-Autoklaven mit Hastelloy C4®-Einsatz, Elektroheizung und Magnetrührer eingefüllt und 100 bar Kohlenmonoxid aufgepreßt. Der Autoklav wird unter Rührung auf 200°C erhitzt und 30 Minuten bei dieser Temperatur gehalten.

Das Gaschromatogramm (GC) der Reaktionslösung zeigt 18,2 Gew.-% Methacrylsäureanhydrid, 29 Gew.-% Essigsäuremethacrylsäure-Mischanhydrid und 12,4 Gew.-% Essigsäureanhydrid an.

Vergleichsbeispiel

Ein Gemisch aus 547,0 mmol Methylmethacrylat, 1,2 mmol Nickeljodid, 50,8 mmol Triphenylphosphin und 100,0 mmol Methyljodid wird in einem 0,35-l-Autoklaven in einer CO-Atmosphäre (100 bar Kaltdruck) für 30 Minuten auf 200°C erhitzt.

Das GC der Lösung läßt 3 Gew.-% 2-Methyl-5-methylenadipinsäure-dimethylester, 0,4 Gew.-% Methacrylsäureanhydrid und 1,5 Gew.-% Essigsäuremethacrylsäure-Mischanhydrid erkennen.

Beispiel 2

547,0 mmol Methylacrylat, 1,0 mmol Rhodium(III)chlorid, 50,8 mmol Triphenylphosphin und 100,0 mmol Methyljodid werden unter CO-Druck

(100 bar Kaltdruck) 30 Minuten auf 200°C erhitzt.

Die GC-Analyse der Lösung gibt 26,2 Gew.-% Acrylsäureanhydrid, 42,5 Gew.-% Essigsäureacrylsäure-Mischanhydrid und 24 Gew.-% Essigsäureanhydrid an.

Beispiel 3

547,0 mmol Methylmethacrylat, 1,0 mmol Rhodium(III)chlorid, 12,0 mmol Molybdänhexacarbonyl, 50,8 mmol Triphenylphosphin und 100 mmol Methyljodid werden in CO-Atmosphäre (100 bar Kaltdruck) 30 Minuten auf 200°C erhitzt.

Laut GC liegen 8,9 Gew.-% Methacrylsäureanhydrid, 26,8 Gew.-% Essigsäuremethacrylsäure-Mischanhydrid und 24,1 Gew.-% Essigsäureanhydrid vor.

Beispiel 4

Ein Gemisch aus 547,0 mmol Methylmethacrylat, 1,0 mmol Rhodium(III)-chlorid, 33,4 mmol Butyrolacton, 6,4 mmol Triphenylphosphin und 66,8 mmol Methyljodid wird in CO-Atmosphäre (100 bar Kaltdruck) 60 Minuten auf 200°C erhitzt.

Das GC der Lösung zeigt 9,3 Gew.-% Methacrylsäureanhydrid, 18,2 Gew.-% Essigsäuremethacrylsäure-Mischanhydrid und 9,4 Gew.-% Essigsäureanhydrid an.

Die GC-Trennung erfolgte bei allen Beispielen in einer Glaskappillarsäule (DB 1701) der Firma J. & W. Scientific Inc., 3871 Security Park Drive, Rancho Cordova (USA).

Beispiel 5

547,0 mmol Methylmethacrylat, 1,0 mmol Platin(II)chlorid, 50,8 mmol Tributylphosphin und 100 mmol Methyljodid werden in CO-Atmosphäre (100 bar Kaltdruck) 30 min auf 200 Grad C erhitzt.

Laut GC liegen 2,8 Gew.-% Methacrylsäureanhydrid, 1,8 Gew.-% Essigsäuremethacrylsäure-Mischanhydrid und 1,4 Gew.-% Essigsäureanhydrid vor.

Beispiel 6

547 mmol Methylmethacrylat, 1,0 mmol Rhodium(III)chlorid, 50,8 mmol Tributylphosphin und 100 mmol Acetylchlorid werden in CO-Atmosphäre (90 bar Kaltdruck) 30 min auf 200 Grad C erhitzt.

Das GC der Lösung zeigt 2,6 Gew.-% Methacrylsäureanhydrid, 2,5 Gew.-% Essigsäuremethacrylsäure-Mischanhydrid und 0,7 Gew.-% Essigsäureanhydrid.

Beispiel 7

547 mmol Methylmethacrylat, 1,0 mmol Rhodium(III)chlorid, 50,8 mmol Tributylphosphin und 100 mmol Ethyljodid werden in CO-Atmosphäre (100 bar Kaltdruck) 30 min auf 200 Grad C erhitzt.

Laut GC der Lösung liegen 13,9 Gew.-% Methacrylsäureanhydrid, 26,6 Gew.-% Essigsäuremethacrylsäure-Mischanhydrid und 20,2 Gew.-% Essigsäureanhydrid vor.

Beispiel 8

547 mmol Ethylmethacrylat, 1,0 mmol Rhodium(III)chlorid, 50,8 mmol

Tributylphosphin und 100 mmol Methyljodid werden in CO-Atmosphäre (100 bar Kaltdruck) 1 Stunde auf 200 Grad C erhitzt.

Laut GC liegen 0,5 Gew.-% Methacrylsäureanhydrid, 1,3 Gew.-% Essigsäuremethacrylsäure-Mischanhydrid und 0,5 Gew.-% Essigsäureanhydrid vor.

Beispiel 9

547 mmol Methylmethacrylat, 1,0 mmol Rhodium(III)jodid, 50,8 mmol Triphenylphosphin und 100 mmol Methyljodid werden in CO-Atmosphäre (100 bar Kaltdruck) 30 min auf 200 Grad C erhitzt.

Das GC-Spektrum zeigt 14,1 Gew.-% Methacrylsäureanhydrid, 26,4 Gew.-% Essigsäuremethacrylsäure-Mischanhydrid und 15,3 Gew.-% Essigsäureanhydrid.

Beispiel 10

547 mmol Methylmethacrylat, 1,0 mmol Palladium(II)chlorid 50,8 mmol Triphenylphosphin und 100 mmol Methyljodid werden in CO-Atmosphäre (100 bar Kaltdruck) 30 min auf 200 Grad C erhitzt.

Das GC-Spektrum zeigt 3,1 Gew.-% Methacrylsäureanhydrid, 0,85 Gew.-% Essigsäuremethacrylsäure-Mischanhydrid und 2,5 Gew.-% Essigsäureanhydrid an.

Beispiel 11

547 mmol Methylmethacrylat, 1,0 mmol Rhodium(III)chlorid, 50,8 mmol Pyridin und 100 mmol Methyljodid werden in CO-Atmosphäre (100 bar Kaltdruck) 30 min auf 200 Grad C erhitzt.

Nach GC der Lösung liegen 2,2 Gew.-% Methacrylsäureanhydrid, 3,5 Gew.-% Essigsäuremethacrylsäure-Mischanhydrid und 1,5 Gew.-% Essigsäureanhydrid vor.

Beispiel 12

547 mmol Methylmethacrylat, 1,0 mmol Rhodium(III)chlorid, 82,0 mmol LiJ, 50,8 mmol Triphenylphosphin und 100 mmol Methyljodid werden in CO-Atmosphäre (100 bar Kaltdruck) 30 min auf 200 Grad C erhitzt.

Das GC der Lösung zeigt 7,7 Gew.-% Methacrylsäureanhydrid, 20,8 Gew.-% Essigsäuremethacrylsäure-Mischanhydrid und 5,6 Gew.-% Essigsäureanhydrid.

Beispiel 13

547 mmol Methylmethacrylat, 2,0 mmol Rhodium(III)chlorid, 70 mmol Tributylphosphin und 100 mmol $J_2$ werden in CO-Atmosphäre (100 bar Kaltdruck) 1 Stunde auf 200 Grad C erhitzt.

Die gaschromatographische Untersuchung des Reaktionsgemisches ergibt, daß es 12,5 Gew.-% Methacrylsäureanhydrid, 10,2 Gew.-% Essigsäuremethacrylsäure-Mischanhydrid und 3,0 Gew.-% Essigsäureanhydrid enthält.

Beispiel 14

Analog Beispiel 1 wird ein Versuch jedoch ohne Phosphinzusatz durchgeführt.

Die Reaktionslösung enthält nach GC 1,0 Gew.-% Methacrylsäureanhydrid,

2,0 Gew.-% Essigsäuremethacrylsäure-Mischanhydrid und 0,7 Gew.-% Essigsäureanhydrid.

Beispiel 15

547 mmol Methylmethacrylat, 1 mmol Rhodium(III)chlorid, 0,5 mmol Platin(II)chlorid 50,8 mmol Tributylphosphin und 100 mmol Methyljodid werden in CO-Atmosphäre (100 bar Kaltdruck) bei 200°C 30 Minuten erhitzt.

Das GC des Reaktionsgemisches zeigt, daß die Lösung 13,8 Gew.-% Methacrylsäureanhydrid, 13,7 Gew.-% Essigsäuremethacrylsäure-Mischanhydrid und 4,4 Gew.-% Essigsäureanhydrid enthält.

Verfahren zur Herstellung von ungesättigten aliphatischen Carbonsäureanhydriden

## Patentansprüche

1. Verfahren zur Herstellung von ungesättigten aliphatischen Carbonsäureanhydriden,

dadurch gekennzeichnet,

daß ein α,ß-ungesättigter Carbonsäureester der allgemeinen Formel

$$H_2C = \overset{\overset{\displaystyle R_1}{|}}{C} - \overset{\overset{\displaystyle O}{\|}}{C} - O - R_2 ,$$

worin

$R_1$ = H oder $CH_3$,
und
$R_2$ = Alkyl mit 1 bis 10 C-Atomen,
Cycloalkyl mit 5 bis 10 C-Atomen,
Alkenyl mit 2 bis 10 C-Atomen, Aryl
oder Aralkyl,
wobei $R_2$ noch inerte Substituenten enthalten kann,

bedeuten,
mit Kohlenmonoxid in Gegenwart eines Katalysatorsystems, das mindestens ein Edelmetall der Gruppe VIII des Periodensystems enthält, und in Gegenwart von Halogen und/oder Halogenverbindungen als Promotoren bei Temperaturen von 70 bis 350°C und Drücken von 1 bis 500 bar umgesetzt wird.

2. Verfahren nach Anspruch 1, dadurch gekennzeichnet, daß die Edelmetalle der Gruppe VIII in den Katalysatorsystemen Rhodium und/oder Palladium und/oder Platin sind.

3. Verfahren nach den Ansprüchen 1 und 2, dadurch gekennzeichnet, daß das Katalysatorsystem noch Komplexliganden, insbesondere Halogen oder Halogenverbindungen und tertiäre organische Phosphorverbindungen bzw. tertiäre organische Stickstoffverbindungen enthält.

4. Verfahren nach Anspruch 1, dadurch gekennzeichnet, daß die Promotoren Brom und/oder Jod und/oder Verbindungen dieser Halogene sind.

5. Verfahren nach den Ansprüchen 1 bis 4, dadurch gekennzeichnet, daß Acrylsäure- und Methacrylsäureester, bei denen $R_2$ ein Alkylrest mit 1 bis 4 C-Atomen ist, mit Kohlenmonoxid umgesetzt werden.

6. Verfahren nach den Ansprüchen 1 bis 5, dadurch gekennzeichnet, daß Acrylsäuremethylester umgesetzt wird.

7. Verfahren nach den Ansprüchen 1 bis 5, dadurch gekennzeichnet, daß Methacrylsäuremethylester umgesetzt wird.

8. Umsetzungsgemisch nach einem der Patentansprüche 1 bis 7, dadurch gekennzeichnet, daß es neben dem reinen α,ß-ungesättigten Carbonsäureanhydrid und dem aus dem Alkoholrest des Esters entstandenen reinen Carbonsäureanhydrid auch das als Primärprodukt gebildete gemischte Anhydrid der α,ß-ungesättigten Carbonsäure und der durch Carbonylierung entstandenen Carbonsäure enthält.